# EUROPEAN PATENT SPECIFICATION

(11) **EP 2 456 517 B1**
(45) Date of publication and mention of the grant of the patent: **06.09.2017**
(21) Application number: 10737707.9
(22) Date of filing: 20.07.2010
(51) Int. Cl.: A61N 1/36, A61N 1/375, A61N 1/08, H04R 25/00, A61F 11/04

(54) **MAGNETIC ATTACHMENT ARRANGEMENT FOR IMPLANTABLE DEVICE**
MAGNETISCHE FIXIERANORDNUNG FÜR EINE IMPLANTIERBARE VORRICHTUNG
CONFIGURATION DE FIXATION MAGNÉTIQUE POUR DISPOSITIF IMPLANTABLE

(30) Priority: 22.07.2009 US 227632 P
(43) Date of publication of application: 30.05.2012
(73) Proprietor: Med-El Elektromedizinische Geräte GmbH, 6020 Innsbruck (AT)
(72) Inventor: BALL, Geoffrey, R., A-6094 Axams (AT); LAMPACHER, Peter, A-6020 Innsbruck (AT); JAMNIG, Bernhard, A-6020 Innsbruck (AT); ZIMMERLING, Martin, A-6082 Patsch (AT); WEIDENHOLZER, Gunther, A-4100 Ottensheim (AT); NAGL, Markus, A-6111 Volders (AT); AMRHEIN, Wolfgang, A-4000 Ottensheim (AT)
(74) Representative: Gillard, Matthew Paul
(86) International application number: PCT/US2010/042598
(87) International publication number: WO 2011/011409

(56) References cited:
- EP-A2- 0 352 954
- WO-A1-2005/123188
- WO-A2-2005/048646
- US-A1- 2004 012 470
- US-A1- 2008 103 350

## Description

### FIELD OF THE INVENTION

The present invention relates to medical implants, and more specifically to a permanent magnet arrangement for use in such implants.

### BACKGROUND ART

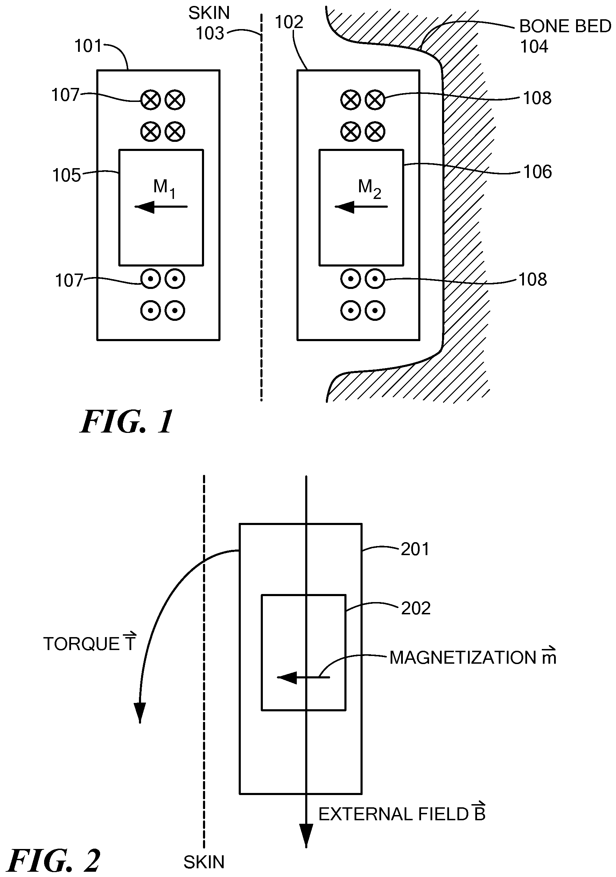

Some hearing implants such as Middle Ear Implants (MEI's) and Cochlear Implants (CI's) employ attachment magnets in the implantable part and an external part to hold the external part magnetically in place over the implant. For example, as shown in Fig. 1, a typical cochlear implant system may include an external transmitter housing **101** containing transmitting coils 107 and an external magnet 105. The external magnet 105 has a conventional coin-shape and a north-south magnetic dipole that is perpendicular to the skin of the patient to produce external magnetic field lines as shown. Implanted under the patient's skin is a corresponding receiver assembly 102 having similar receiving coils 108 and an implanted internal magnet 106. The internal magnet 106 also has a coin-shape and a north-south magnetic dipole that is perpendicular to the skin of the patient to produce internal magnetic field lines **108** as shown. The internal receiver housing 102 is surgically implanted and fixed in place within the patient's body. The external transmitter housing **101** is placed in proper position over the skin covering the internal receiver assembly 102 and held in place by interaction between the internal magnetic field lines and the external magnetic field lines. Rf signals from the transmitter coils 107 couple data and/or power to the receiving coil **106** which is in communication with an implanted processor module (not shown).

One problem arises when the patient undergoes Magnetic Resonance Imaging (MRI) examination. Interactions occur between the implant magnet and the applied external magnetic field for the MRI. As shown in Fig. 2, the direction magnetization 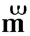 of the implant magnet **202** is essentially perpendicular to the skin of the patient. Thus, the external magnetic field 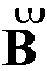 from the MRI may create a torque 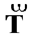 on the internal magnet **202,** which may displace the internal magnet **202** or the whole implant housing **201** out of proper position. Among other things, this may damage the adjacent tissue in the patient. In addition, the external magnetic field 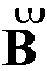 from the MRI may reduce or remove the magnetization 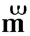 of the implant magnet **202** so that it may no longer be strong enough to hold the external transmitter housing in proper position. The implant magnet **202** may also cause imaging artifacts in the MRI image, there may be induced voltages in the receiving coil, and hearing artifacts due to the interaction of the external magnetic field 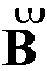 of the MRI with the implanted device. This is especially an issue with MRI field strengths exceeding 1.5 Tesla.

Thus, for existing implant systems with magnet arrangements, it is common to either not permit MRI or at most limit use of MRI to lower field strengths. Other existing solutions include use of a surgically removable magnets, spherical implant magnets (e.g. U.S. Patent 7,566,296), and various ring magnet designs (e.g., U.S. Provisional Patent 61/227,632, filed July 22, 2009). Among those solutions that do not require surgery to remove the magnet, the spherical magnet design may be the most convenient and safest option for MRI removal even at very high field strengths. But the spherical magnet arrangement requires a relatively large magnet much larger than the thickness of the other components of the implant, thereby increasing the volume occupied by the implant. This in turn can create its own problems. For example, some systems, such as cochlear implants, are implanted between the skin and underlying bone. The "spherical bump" of the magnet housing therefore requires preparing a recess into the underlying bone. This is an additional step during implantation in such applications which can be very challenging or even impossible in case of very young children.

Various complicated arrangements of magnetic elements have been described for use in therapeutic applications; see for example, U.S. Patent 4,549,532 and U.S. Patent 7,608,035. However, there is no suggestion that such therapeutic arrangements might potentially have any utility for magnetic attachment applications such as those described above.

US 2004/0012470 describes an MRI safe implant cylindrical magnet arrangement according to the preamble of claim 1.

### SUMMARY OF THE INVENTION

The object of the present invention is achieved with an implantable device according to claim 1. According to the invention, the magnetic sections may include an inner center disc having an inner magnetic orientation in an inner magnetic direction, and an outer radial ring having an outer magnetic orientation in an outer magnetic direction opposite to the inner magnetic direction. Or the magnetic sections may include multiple wedge sections formed together into a cylinder shape wherein adjacent wedge sections have opposing magnetic orientations in opposite magnetic directions. Or the magnet arrangement may include an inner center disc surrounded by outer radial ring having a plurality of wedge sections wherein adjacent wedge sections have opposing magnetic orientations in opposite magnetic directions.

Many embodiments also have an implant signal coil within the housing surrounding the implant magnet arrangement for receiving an implant communication signal. In some embodiments, there may be multiple implant magnet arrangements. There may also be a similar external housing having a corresponding magnet arrangement. The implantable electronic system may be, for example, a cochlear implant system, a middle ear implant system, or a bone conduction hearing implant system.

### BRIEF DESCRIPTION OF THE DRAWINGS

Figure 1 shows a portion of a typical idealized cochlear implant which may be used in embodiments of the present invention.
Figure 2 shows effects of an external magnetic field on an implanted portion of an implanted device which may be used in embodiments of the present invention.
Figure 3 A-B shows an implant magnet arrangement according to embodiments of the present invention.
Figure 4 shows how an embodiment of an implant magnet arrangement cooperates with a typical external device.
Figure 5 shows how an embodiment of an implant magnet arrangement cooperates with another corresponding external magnet arrangement.
Figure 6 shows an embodiment of an implant magnet having magnetically alternating pie-shaped magnetic sections.
Figure 7 shows another embodiment similar to the one in Fig. 6 with an inner center disk.

### DETAILED DESCRIPTION OF SPECIFIC EMBODIMENTS

Various embodiments of the present invention are directed to an improved magnet arrangement for implantable devices in the form of a cylindrical magnet having multiple adjacent magnetic sections wherein at least two of the magnetic sections have opposing magnetic orientations in opposite magnetic directions.

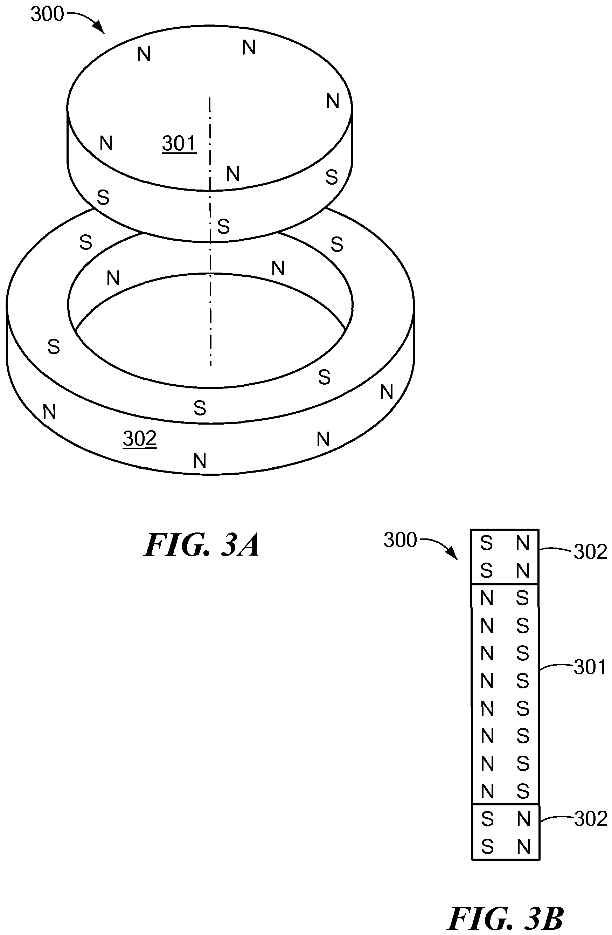

Figure 3A shows an exploded elevated view and Figure 3B shows a side view of an implant magnet arrangement **300** according to embodiments of the present invention. An implantable housing (e.g., implant housing **102**) contains a portion of an implantable electronic system. The implantable electronic system may be, for example, a cochlear implant system, a middle ear implant system, or a bone conduction hearing implant system. A cylindrical implant magnet arrangement **300** within the housing includes an inner center disc section **301** having an inner magnetic orientation in an inner magnetic direction, and an outer radial ring section **302** having an outer magnetic orientation in an outer magnetic direction opposite to the inner magnetic direction.

With such an arrangement, the net magnetic field of the implant magnet arrangement **300** is much less than in the conventional cylindrical magnet of the prior art, while locally the magnetic fields are still effectively strong near the inner center disc section **301** and the outer radial ring section **302** so that there is no overall loss in the retention force of the implant magnet arrangement **300.** Such a reduced net magnetic field of the implant magnet arrangement **300** also avoids the prior problems of the net magnetic fields adversely interacting with the implant signal coil and its communications signal and reduces the torque and imaging problems of the prior art with regards to MRI procedures. Moreover, the greater specificity of the magnetic structures of the implant magnet arrangement **300** compared with a simple disk magnet also provides improved centering capability with regards to the external component housing.

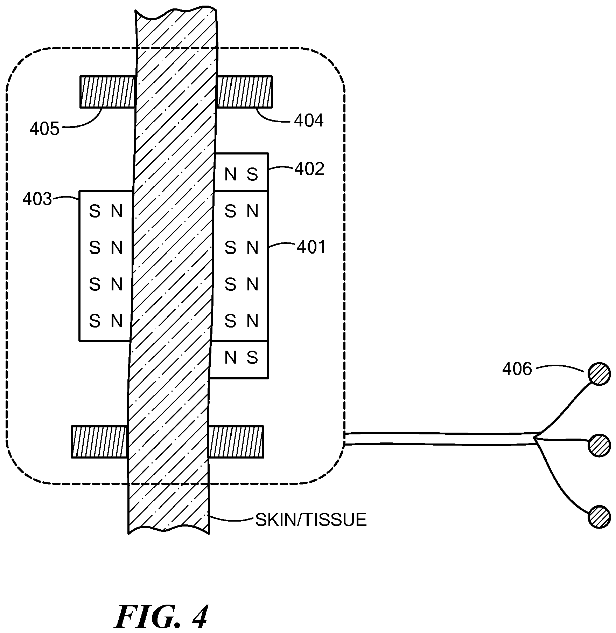

Figure 4 shows how an embodiment of an implant magnet arrangement cooperates with a typical external device. A conventional cylindrical external magnet **403** interacts with an implant magnet having an inner center disc section **401** and an outer radial ring section **402** according to an embodiment of the invention. In this case, the external magnet **403** is similar in diameter to the inner center disc section **401** of the implant magnet so that their respective magnetic fields interact to provide the desired retention force to hold the external device in proper operating position. This allows external signal coil **405** to couple an implant communications signal containing data and power through to a corresponding implant coil **404.** The implant communications signal received by the implant coil **404** then is coupled to other elements **406** of the implant system such as an implant processor of a cochlear implant, bone conduction transducer, or middle ear transducer. In some embodiments, there may be multiple implant magnet arrangements and corresponding external magnets.

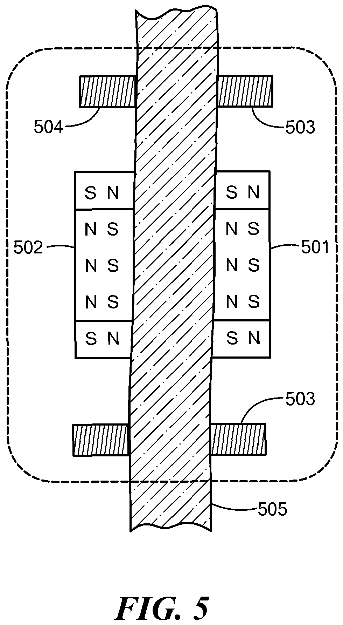

Figure 5 shows how an embodiment of an implant magnet arrangement cooperates with another corresponding external magnet arrangement. In this case, the external magnet **502** also has inner and outer sections that correspond to similar sections of the implant magnet **501** to cooperate to hold the external device in proper operating position. In some embodiments, there may be multiple implant magnet arrangements. This allows an external signal coil **504** to couple an implant communications signal containing data and power across the skin **505** to a corresponding implant coil **503** for use by other elements of the implant system.

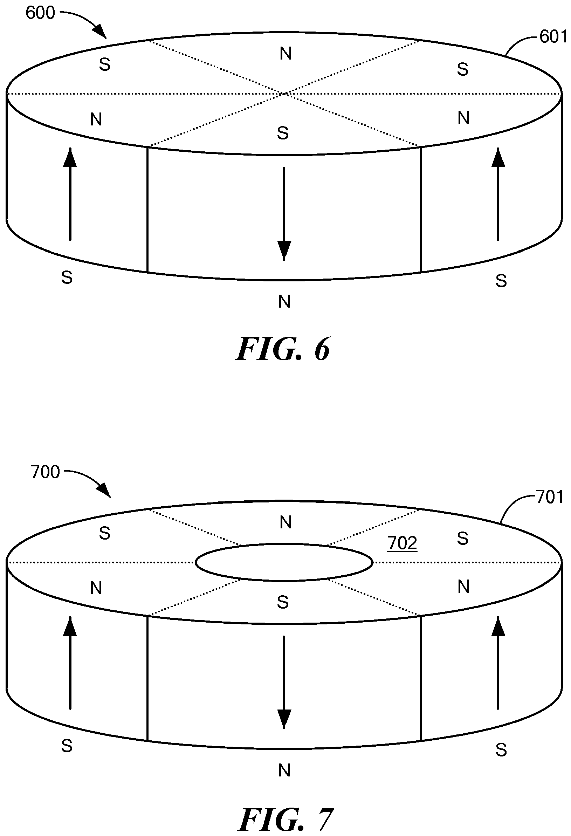

Figure 6 shows another arrangement not according to the present invention where an implant magnet arrangement **600** includes axial magnetized wedge sections 601 with adjacent wedge sections having diametrically opposed magnetic orientation. The implant magnet arrangement **600** in Fig. 6 shows six magnetized wedge sections **601,** but other embodiments may have different numbers of wedge sections so long as the overall net magnetic field of the arrangement as a whole is minimized. In addition, Figure 7 shows another embodiment with an inner center disk **701** which may or may not be magnetized, surrounded by an outer radial ring **702** which is sub-divided into magnetized partial wedge sections **703** where adjacent wedge sections are oppositely magnetized. In such arrangements (or indeed, many of the above embodiments), between the individual magnetized sections there also may be narrow unmagnetized transition elements.

Embodiments such as the one shown in Figs. 6 and 7 allow the external housing to be attached on the skin at a fixed specified angle, which can be useful for ensuring proper alignment of directional microphones. Of course, for some applications this might be seen as a drawback in that the external housing can be only be fixed at a limited number of specific angles depending on the numbers of wedge sections. For example, if the implant magnet arrangement **600** has four axial magnetized wedge sections 601, then the external part can only be rotated at an angle of 180°. With six magnetized wedge sections **601,** the rotation angle is 120°.

Embodiments of the present invention such as those described above can be easily and directly implemented in existing products with corresponding size and geometry replacement magnets, either for the implanted magnet and/or the external magnet. Embodiments may usefully contain permanent magnetic material and/or ferromagnetic material as well as other structural materials. These include without limitation magnetic ferrite materials such as Fe₃O₄, BaFe₁₂O₁₉ etc., compound materials such as plastic bonded permanent magnetic powder, and/or sintered material such as sintered NdFeB, SmCo, etc. Selection of the proper materials and arrangements may help avoid or reduce undesired eddy currents.

## Claims

1. An implantable device comprising:
an implant housing containing a portion of an implantable electronic system; and
a cylindrical implant magnet arrangement within the housing **characterised in that** the cylindrical implant magnet arrangement includes:
i. an inner center disc having an inner magnetic orientation in an inner magnetic direction, and
ii. an outer radial ring having an outer magnetic orientation in an outer magnetic direction opposite to the inner magnetic direction.

2. An implantable device according to claim 1, further comprising:
an implant signal coil within the housing, surrounding the implant magnet arrangement for receiving an implant communication signal.

3. An implantable device according to claim 1, wherein the implantable electronic system includes a cochlear implant system.

4. An implantable device according to claim 1, wherein the implantable electronic system includes a middle ear implant system.

5. An implantable device according to claim 1, wherein the implantable electronic system includes a bone conduction hearing implant system.

6. An implantable device according to claim 1, wherein the cylindrical implant magnet arrangement is for cooperating with an external magnetic arrangement of the implantable electronic system to hold the external magnetic arrangement in its operating position.

## Patentansprüche

1. Implantierbare Vorrichtung, umfassend:
ein Implantat-Gehäuse enthaltend einen Bereich eines implantierbaren elektronischen Systems; und
eine zylindrische Implantat-Magnetanordnung in dem Gehäuse,
**dadurch gekennzeichnet, dass** die zylindrische Implantat-Magnetanordnung umfasst:
i. eine innere Zentrumsscheibe mit einer inneren Magnetorientierung in einer inneren Magnetrichtung; und
ii. einen äußeren radialen Ring mit einer äußeren Magnetorientierung in einer zu der inneren Magnetrichtung entgegengesetzten äußeren Magnetrichtung.

2. Implantierbare Vorrichtung gemäß Anspruch 1, ferner umfassend:
eine Implantat-Signalspule in dem Gehäuse, die die Implantat-Magnetanordnung umschließt, um ein Implantat-Kommunikationssignal zu empfangen.

3. Implantierbare Vorrichtung gemäß Anspruch 1, wobei das implantierbare elektronische System ein Cochlear-Implantatsystem umfasst.

4. Implantierbare Vorrichtung gemäß Anspruch 1, wobei das implantierbare elektronische System ein Mittelohr-Implantatsystem umfasst.

5. Implantierbare Vorrichtung gemäß Anspruch 1, wobei das implantierbare elektronische System ein Knochenleitungs-Hörimplantatsystem umfasst.

6. Implantierbare Vorrichtung gemäß Anspruch 1, wobei die zylindrische Implantat-Magnetanordnung vorgesehen ist für ein Zusammenwirken mit einer externen Magnetanordnung des implantierbaren elektronischen Systems, um die externe Magnetanordnung in ihrer Betriebsposition zu halten.

## Revendications

1. Dispositif implantable comportant :
- un boîtier d'implant logeant une partie d'un système électronique implantable et un montage d'aimant d'implant, cylindrique, dans le boîtier,
dispositif **caractérisé en ce que**
le montage d'aimant d'implant cylindrique comporte :
i. un disque central intérieur ayant une orientation magnétique intérieure dans la direction magnétique intérieure, et
ii. un anneau radial extérieur ayant une orientation magnétique extérieure dans la direction magnétique extérieure à l'opposé de la direction magnétique intérieure.

2. Dispositif implantable selon la revendication 1,
**caractérisé en ce qu'**il comporte en outre
une bobine de signal d'implant dans le boîtier entourant le montage d'aimant d'implant pour recevoir un signal de communication d'implant.

3. Dispositif implantable selon la revendication 1,
**caractérisé en ce que**
le système électronique implantable comporte un système d'implant cochléaire.

4. Dispositif implantable selon la revendication 1,
**caractérisé en ce que**
le système électronique implantable comporte un système d'implant d'oreille moyenne.

5. Dispositif implantable selon la revendication 1,
**caractérisé en ce que**
le système électronique implantable comporte un système d'implant auditif à conduction osseuse.

6. Dispositif implantable selon la revendication 1,
**caractérisé en ce que**
le montage d'aimant d'implant, cylindrique coopère avec un dispositif magnétique externe du système électronique implantable pour tenir le dispositif magnétique externe dans sa position de fonctionnement.
